# FASCICULE DE BREVET EUROPEEN

(11) **EP 1 542 763 B1**
(45) Date de publication et mention de la délivrance du brevet: **19.07.2006**
(21) Numéro de dépôt: 03798224.6
(22) Date de dépôt: 24.09.2003
(51) Int. Cl.: A61N 1/32

(54) **DISPOSITIF POUR LE BLANCHIMENT UTILISANT DES MOYENS ÉLECTRO-OPTIQUES ET CHIMIQUES, NOTAMMENT DANS LE DOMAINE MÉDICAL ET DENTAIRE**
BLEICHVORRICHTUNG MIT ELEKTROOPTISCHEN UND CHEMISCHEN MITTELN, INSBESONDERE ZUR VERWENDUNG IN DER MEDIZIN UND ZAHNMEDIZIN
BLEACHING DEVICE EMPLOYING ELECTRO-OPTICAL AND CHEMICAL MEANS, WHICH IS INTENDED, IN PARTICULAR, FOR USE IN THE MEDICAL AND DENTAL FIELD

(30) Priorité: 24.09.2002 FR 0211769
(43) Date de publication de la demande: 22.06.2005
(73) Titulaire: Duret, François, F-11560 Fleury d'Aude (FR)
(72) Inventeur: Duret, François, F-11560 Fleury d'Aude (FR)
(74) Mandataire: Joly, Jean-Jacques
(86) Numéro de dépôt international: PCT/FR2003/002814
(87) Numéro de publication internationale: WO 2004/028626

(56) Documents cités:
- WO-A-01/80945
- WO-A-01/91853
- WO-A-98/06456
- US-A- 5 246 019
- US-B1- 6 385 487

## Description

La présente invention a pour objet un dispositif pour le blanchiment des corps assurant leur intégrité structurale, applicable notamment dans les domaines industriels pour les tissus, le domaine médical pour les ongles et les poils et le domaine dentaire pour les dents, comportant un créateur de champ électrophorétique entraînant directement, ou par l'intermédiaire d'un produit électriquement actif, les molécules actives blanchissantes vers leurs cibles, les produits dégradés ou non responsables de la coloration vers l'extérieur et éventuellement une ou des molécules actives capables de reconstituer le corps ayant subit l'action des produits de blanchiment dans le corps diminuant ainsi toute dégradation à court ou moyen terme que pourraient provoquer les applications chimiques des produits actifs et le retrait des molécules colorantes et comportant aussi éventuellement une source thermique et/ou photonique activant ou accélérant l'activation des produits mis en oeuvre pour le blanchiment, le tout sous le contrôle de capteur électrique ou colorimétrique.

Blanchir une dent, où plus exactement lui donner sa couleur naturelle d'origine, spécifique du patient, est un acte couramment pratiqué dans les cabinets dentaires depuis plus de 100 ans et très important pour l'équilibre psychologique des malades.

Une très bonne explication de cet acte esthétique a été donnée, et fait référence en la matière : le supplément spécial du JADA (Journal American Dental Assos.) d'avril 1997, n°128 (supplément) pp S1 à S64 intitulé « non restorative treatment of discolored teeth, reports for an international symposium » et résumant le congres de Chapel Hill , North Carolina des 25 et 26 septembre 1996. Une actualisation peut être trouvée dans le CRA (Clinical research Associates newsletter) Volume 24 , issue 4 de avril 2000 ou encore plus récemment dans "Incidence of tooth sensitivity after home whitening treatment" par Jorgensen and coll. JADA , Aout 2002 , Vol. 133 pp 1076-1082.

Il ressort de ces travaux qu'il existe aujourd'hui trois grandes méthodes de blanchiment utilisées dans les cabinets dentaires et médicaux :
- une méthode mécanique consistant à associer au détartrage des dents par des moyens mécaniques (manuel et ultrason) des pâtes abrasives de polissage.
- une méthode chimique, en général suivant la précédente, et consistant à appliquer sur la dent un produit capable de supprimer les dépôts superficiels comme ceux dus au thé ou au café. Ces produits sont souvent à base de carboxyde ou de peroxyde, à très faible concentration et peuvent être utilisés par le patient lui-même à domicile.
- une méthode chimique plus invasive et ayant des concentrations plus élevées de produit à base de peroxyde, nécessitant souvent l'intervention du chirurgien dentiste compte tenu des risques encourus par le patient s'il n'appliquait pas sa thérapeutique dans les règles de l'art dentaire et médical, et permettant de réduire la coloration des dents à l'intérieur de l'organe dentaire lui-même. Récemment cette méthode a été modifiée et réduite en action pour être utilisable par le patient lui-même à son domicile (home kit) sous le couvert d'un contrôle médical régulier.

Malheureusement et très rapidement les praticiens comme leurs patients se sont rendus compte que :
- le temps d'application était très élevé et nécessitait l'immobilisation du patient pendant plus de 5 minutes par dent ou 20 minutes par hémi-arcades.
- que de ce fait le coût de l'intervention était pénible et prohibitif !

C'est pour cette raison qu'ont été développés des produits réagissant plus rapidement en étant activés par la lumière ou la chaleur. Cette méthode permit de réduire par quatre le temps imparti à l'acte médical de blanchiment.

S'appuyant sur ces résultats prometteurs un certain nombre de produits dits photosensibles ont été mis sur le marché et utilisés abondamment avec succès, associant perborate et peroxyde d'hydrogène ou carbamide de peroxyde activé par la camphoroquinone elle-même photosensible entre 400 et 500 nm. Ces produits sont issus des techniques développées et présentées originellement par CORCORAN et ZILLICH (1974) et par RENNEBOOG (1989). Ces études mettent en évidence le rôle de la chaleur et du rayonnement apporté par les lampes halogènes dans l'activation des produits de blanchiment.

Il existe donc aujourd'hui sur le marché de nombreux produits de blanchiment utilisables directement par le patient chez lui ou applicable à plus forte concentration par les dentistes. Ces produits agissent directement ou après activation par la lumière ou la chaleur. Ils utilisent en grande majorité comme formule de base le peroxyde d'hydrogène à environ 35 % tel que l'avait décrit pour la première fois HALON en 1884.

Pour activer encore plus la réaction et diviser encore par deux le temps déjà considérablement réduit, il a été demandé de développer des lampes encore plus puissantes et c'est à cet effet qu'a été inventée et mise au point la lampe plasmatique à arc xénon "Apollo 95^{E"}, brevets FR 2.773.986 et FR 2.782.000, qui comportait une fonction « blanchiment » et qui supposait une action de l'ordre de 30 secondes sur le produit placé au contact de la dent. Ce produit est souvent sous forme de gel maintenu dans une gouttière transparente. Un exemple type a été vendu longtemps sous le nom de « Apollo secret whitening kit » (DMDS Corp. Los Angeles, USA).

Certes les résultats obtenus ont été spectaculaires et de nombreux fabricants ont suivi cette technologie. Pourtant ce procédé, même s'il diminuait considérablement le temps, avait de nombreuses limites.

Il a en effet été montré que:
- avec certain produit l'action de la lampe, donc son efficacité, était non seulement due aux émissions photoniques mais aussi thermiques et c'est sous ces deux effets que ces produits étaient activés.
- le coût de ces lampes étant très élevé, le traitement restait relativement cher,
- ces méthodes nécessitant des concentrations élevées de peroxyde, elles empêchaient leur utilisation à domicile par le patient.
- l'élévation thermique observée dans la dent était disproportionnée par rapport à l'activation du produit et pouvait même être dangereuse si l'action était trop longue.
- il était impossible de contrôler correctement la valeur thermique au niveau même de la dent au risque de provoquer des désordres importants dans la santé de la pulpe dentaire elle-même. De plus le mouvement même de la main pouvait entraîner des variations de localisation du point d'impact lumineux ou thermique.
- en remplaçant la lumière émise par les lampes à forte émission thermique comme les arcs xénon ou les lampes halogènes, par des lampes dites froides telles que celles décrites dans les documents FR 2.805.148 et FR 2.818.892, l'effet thermique était supprimé ce qui permettait à l'opérateur d'augmenter sans risque son temps d'action pour activer les produits photosensibles mais parallèlement l'obligait à nouveau à une longue exposition du fait de la suppression de la source de chaleur.
- si l'on utilise une lumière dispersée sur toute l'arcade (par exemple sous forme de gouttière lumineuse) le temps se trouve à nouveau diminué mais pas plus qu'en utilisant les fortes puissances.
- les doses mises en oeuvre pour le traitement chez le dentiste (30% de peroxyde) comme chez le patient (8 - 10% de peroxyde) sont extrêmement élevées par rapport à l'objectif visé et explique les effets secondaires observés (douleurs dentaires au froid...).
- il existe de nombreuses récidives car les molécules responsables de la coloration sont modifiées, voir coupées, mais jamais réellement évacuées du site. Le fait qu'elles subsistent permet la recombinaison de liaisons chimiques initiales expliquant la nécessité de retraiter suivant des périodes de plus en plus rapprochées.
- enfin l'action agressive du peroxyde au contact de la gencive oblige toujours le praticien comme le patient à de nombreuses précautions pour éviter les brûlures durant et après le traitement.

De plus à aucun moment n'était résolu un problème fondamental qui a justifié la mise en surveillance des produits de blanchiment et en particulier du peroxyde par toutes les commissions de sécurité dans le domaine de la santé de la CE à la FDA : quel est le degré de dégradation de la dent elle-même sous l'effet de ces produits, qu'ils soient appliqués massivement à haute dose, ou lentement à demeure chez le patient lui-même ? Les douleurs post opératoires sont-elles le résultat d'une dégradation de la dent telle que l'ont affirmé de nombreux auteurs ? Comment corriger certaines décalcifications significatives observées chez les patients après des traitements appliqués de manière erronée ?

En fin de compte s'il est possible au travers des techniques connues de permettre une action plus rapide des produits de blanchiment, il n'existe aucune méthode permettant de contrôler, à faible coût, l'activation des composants chimiques de blanchiment et de corriger la dégradation de la dent suite à leurs effets.

Plus grave encore est le devenir des molécules responsables de la coloration. En effet après un traitement essentiellement basé sur la division de grosses molécules colorées en plus petites, le résidu de cette réaction reste, avec l'agent actif, à l'intérieur de l'organe dentaire continuant sournoisement son effet et limitant significativement la pénétration du fluor ou du calcium.

En plus des conséquences de cet encombrement stérique, nous voyons bien que l'arrivée sur le site d'action intradentaire de la molécule de blanchiment se fait par pure perméabilité passive expliquant sans doute, sa faible pénétration, le temps mis pour permettre son action, l'absence de contrôle possible de la réaction par l'opérateur mais aussi une méconnaissance totale de sa concentration réelle dans la zone d'action, c'est à dire sur les molécules responsables de la coloration dans le tissu dentaire.

Enfin l'absence de référence objective et peu coûteuse du départ et du suivi de l'évolution du blanchiment de la dent rend parfaitement subjectif son estimation.

Le document WO-A-98/06456 montre un dispositif selon le préambule de la revendication 1.

La présente invention a pour but de résoudre les inconvénients précités en proposant un ensemble complet de blanchiment composé d'un système électrochimique, d'application notamment dans les domaines dentaire et médical, permettant, grâce à la création d'un champ électrophorétique, une pénétration forte, rapide et directive des agents de blanchiment, leur retrait dans les mêmes conditions accompagnées des molécules responsables de la coloration et une pénétration efficace et contrôlée des agents stabilisateurs du cristal d'apatite, constituant de base de la dent, tel le fluor, mais aussi grâce à son système optique à base de lumière de permettre l'activation des produits de blanchiment photo-sensible en diminuant l'effet chaleur tout en augmentant l'effet photonique grâce à un choix de longueur d'onde se situant aux alentours de 400-500 nm et, enfin, grâce à un ensemble électro-optique le contrôle de l'évolution de la coloration par des méthodes objectives basées sur la spectrocolorimètrie.

La présente invention a pour but de répondre à ces problèmes en proposant une solution modulable, peu onéreuse et utilisable aussi bien dans un cabinet dentaire que, sous une forme simplifiée, chez le patient lui-même.

En particulier elle répond aux nombreux problèmes évoqués précédemment car:
- par les moyens mis en oeuvre, le dispositif offre une source énergétique naturelle d'activation des composants responsables du blanchiment qui est l'effet ohm dû au passage du courant électrophorétique dans un gel, l'appoint apporté par des sources de chaleur électrique ou encore l'adjonction d'une émission photonique (LEDs).
- le coût d'un tel système, qui permet d'éviter l'utilisation des lampes, est extrêmement faible car une source électrophorétique n'est qu'une alimentation électrique. Ce coût devient très faible si on utilise la même alimentation pour une brosse à dent électrique et le dispositif de la présente invention.
- les moyens de contrôle de l'énergie électrophorétique, fournis par une alimentation simple semblable à une alimentation de brosse à dent électrique, permet son utilisation au domicile du patient. La communication par modem permet au dentiste de suivre l'évolution du traitement via Internet..
- l'élévation thermique provoquée par le dispositif permet d'activer les molécules de blanchiment avant de les déplacer vers le centre de la dent.
- la valeur thermique peu être totalement contrôlable grâce à un dispositif feed back indépendante de l'opérateur.
- si l'on utilise des LEDs, il n'est pas nécessaire d'augmenter le temps par rapport aux lampes à forte émission thermique car à l'effet photonique se joint l'effet thermique naturel accompagnant tout courant électrophorétique.
- les LEDs étant minuscules, elles peuvent être placées contre le site opératoire, évitant l'utilisation d'une lampe avec fibres, ce qui réduit considérablement le coût du dispositif.
- les doses misent en oeuvre pour le traitement chez le dentiste comme à domicile sont très réduites pour un effet identique et très supérieur à doses égales car les molécules actives sont apportées sur le site coloré et les molécules responsables de cette coloration en sont évacuées par les courants électrophorétiques.
- l'évacuation des molécules colorées hors de la dent réduit les récidives.
- la diminution des doses pour un effet similaire permet de réduire les effets secondaires ou indésirables comme l'action caustique sur la gencive.

A cet effet, la présente invention concerne un dispositif d'activation de molécules réactives responsables du blanchiment et d'amplification sélective de leurs mouvements vers l'intérieur de la dent, du mouvement inverse des produits résultants de leur action interne et de leur remplacement, par un mouvement lui aussi captivé par le dispositif, des molécules stabilisant et renforçant la structure de la dent le tout sous le contrôle d'un système de mesure de leur efficacité.

Ainsi le dispositif selon l'invention est defini par la revendication 1. Les revendications 2-17 concernent des modes de realisation preférés du dispositif selon l'invention. Le dispositif comprend :
- une unité centrale permettant de contrôler les paramètres définissant la dynamique thermique (comme l'intensité, la variation, la vitesse ou l'accélération), la dynamique photonique (comme la puissance et la longueur d'onde), la dynamique électrophorétique (comme l'intensité, la puissance, la fréquence, le profil et la modulation mais aussi un système de contrôle par feed back de l'action en cours ou obtenue), le temps, le stockage, la lecture et la transmission des données sur différents supports
- un ensemble extrabuccal, essentiellement électro-opto-numérique inclus ou non dans l'unité centrale et renfermant :
- un système électronique créant un champ de polarisation nous permettant d'obtenir un champ électrique, électromagnétique ou électrophorétique entre le gel déposé sur la dent et l'intérieur de la dent et ayant pour fonction d'activer et de diriger un flux ionique direct.

Par action directe nous entendons que le champ électrique que nous avons créé agit directement sur les charges présentes (et globales) des molécules qui nous intéressent dans le blanchiment. Nous aurons donc un champ électrique ou magnétique agissant directement sur les charges positives ou négatives des molécules actives, comme le peroxyde responsable du blanchiment, les entraînant vers l'intérieur de la dent pour qu'elles y pénètrent rapidement et fortement. Nous aurons aussi un mouvement inverse, simultané ou non, des molécules colorantes, entières ou dégradées, par les agents de blanchiment afin qu'elles ne restent pas dans l'émail et qu'elles ne risquent pas de se recombiner. Nous aurons enfin un dernier mouvement, combiné ou non avec les agents de blanchiment, des ions reconstituants de la dent vers l'intérieur de l'émail (par exemple l'ion négatif fluor à des doses de 1,1 ppm).

Cette action peut être indirecte si les molécules à déplacer ne sont pas électriquement actives. Dans ce cas, ce ne sont plus les molécules agissantes (peroxyde, colorant et fluor...) qui sont entraînées par le champ électrique, mais un produit complémentaire sensible au champ électrique et capable par ses caractéristiques électriques d'être entraîné par ce champ, et par ses caractéristiques chimiques, de se fixer sur les molécules agissantes ou simplement de les entraîner sans liaison chimique mais par entraînement passif comme tout fluide en mouvement est capable d'entraîner des particules.

Il est à préciser que l'incorporation d'ions stabilisateurs et renforçateurs de la dent comble les éventuels vides résultant de l'action du blanchiment.

Selon une caractéristique additionnelle du dispositif selon l'invention, il comporte un dispositif activant les produits photosensibles grâce à un système de lumière à LEDs, halogène ou à arc, associé à un système thermique complémentaire dans le cas où est utilisée de la lumière froide (LED) et/ou si les produits actifs sont thermosensibles. Le transport de l'énergie nécessaire jusqu'à la zone à blanchir se fait à l'aide de fibre s'il s'agit d'énergie lumineuse, de fluide s'il s'agit l'énergie thermique et de fils s'il s'agit d'énergie électrique.

Selon une autre caractéristique additionnelle du dispositif selon l'invention, il comporte un capteur spectro ou colorimétrique capable de stocker et d'indiquer par principe comparatif, les réglages spécifiques et l'évolution de la coloration des dents. Ce spectrocolorimètre de conception élémentaire et peu coûteuse, permet de mesurer avant et après traitement le changement progressif de la coloration du gel se chargeant de produits dégradés et/ou de la dent traitée s'en trouvant libérée.

Le dispositif comprend également un ensemble endobuccal renfermant :
- une gouttière, généralement transparente, capable de contenir les produits de blanchiment comme les solutions de peroxyde, les produits colorants dégradés par le peroxyde, et les gels fluorés (par exemple). Ces gouttières peuvent être standards ou individuelles pour chaque patient et/ou chaque type de produit, en particulier, lors d'action indirecte.
- éventuellement une gouttière incluse dans la première ou adaptable sur celle-ci, portant les sources de lumières (LED par exemple) ou conduisant la lumière (halogène ou plasmatique) sur toutes ou partie de l'arcade,
- une connexion électrophorétique assurant, suivant sa polarisation, l'évacuation rapide des composants dégradés sous l'action de la lumière ou du peroxyde activé, et à l'inverse, la pénétration des produits de blanchiment ou du gel chargé d'éléments reconstitutifs de la dent,
- éventuellement une source thermique, comme par exemple, des filaments électriques permettant une élévation contrôlée de la température au niveau des dents à traiter.
- un gel renfermant les produits de blanchiment et/ou les ions reconstitutifs utilisés pour la conduction du courrant dans la gouttière de l'extérieur vers l'intérieur de la dent, et, éventuellement, un autre type de gel de polarisation inverse ou identique au précédent, mais dont la polarisation a été inversée et chargée de recevoir, et éventuellement de piéger, les molécules colorantes déplacées hors de la dent. Ce gel constitue le premier pôle de polarisation.

Le dispositif comprend également un élément polaire, sorte de poignée tenue dans la main ou dans la dent ou encore une surface souple conductrice appliquée dans le dos du patient et permettant d'apporter la polarité inverse dans le corps humain à celle appliquée dans le gel de la gouttière endobuccale. Nous savons aujourd'hui que cette polarité sera transmise dans la pulpe dentaire grâce aux liquides présents dans le corps humain. Le corps humain est donc le deuxième pôle créant le champ électrophorétique avec le gel de la gouttière.

Cette invention consiste aussi à associer à un ensemble cohérent de procédés de blanchiment connus, les mouvements électrophorétiques décrits ici et d'en contrôler les résultats obtenus grâce à un système de surveillance simple comme par exemple par colorimétrie.

L'invention consiste donc à blanchir plus rapidement les dents pour réduire le coût du traitement en transportant de manière active les produits de blanchiment très près des molécules responsables de leur coloration, à assurer une évacuation nouvelle, unique et réelle, des composants dégradés, et à renforcer la structure apatite de l'émail, grâce à l'apport actif d'ions comme le calcium ou le fluor sur le site. Cette conception nouvelle et unique est mise en oeuvre grâce à l'utilisation de courants électrophorétiques conjugués à un système d'activation thermique ou photonique favorisant et pouvant résulter de l'électrophorèse.

Nous savons aussi qu'un courant d'électrophorèse fait augmenter la température du milieu, lors du passage du courant sans autre intervention extérieure. Avantageusement, la présente invention, consiste donc à choisir un gel porteur des principes actifs de blanchiment capable d'optimiser l'effet ohm bien connu, accompagnant le passage du courant, et activant du même coup les molécules actives de peroxyde... en évitant l'utilisation de sources extérieures thermiques ou photoniques.

Avantageusement, et selon une caractéristique additionnelle du dispositif selon l'invention, si l'élévation normale de la température n'est pas suffisante, il comprend une gouttière renfermant un filament unique servant à la fois de pôle électrophorétique, et de résistance électrique permettant l'élévation thermique lors du passage du courant, créant ainsi, la source de chaleur. Nous savons, en effet, que tout passage de courant s'accompagne d'un effet ohm. Il est donc ici proposé d'utiliser un fil de polarisation ayant en plus une élévation thermique rapide et conséquente lors du passage du courant, même faible, et permettant ainsi de renforcer l'effet chimique et électrophorétique, déjà décrit, d'un effet thermique activant les molécules en augmentant la vibration énergétique, les rendant plus réactives.

Avantageusement et selon une autre caractéristique additionnelle du dispositif selon l'invention, le filament électrique sera d'importance différente ou de matériaux différents, suivant l'effet désiré et, par exemple, et sans que ceci soit limitatif, il sera plus important dans le cas d'une utilisation sous le contrôle d'un professionnel, comme un chirurgien dentiste, afin d'obtenir une élévation thermique plus forte et plus rapide. Il sera moins puissant dans le cas d'une utilisation privée à domicile afin d'éviter tout risque.

Selon une autre caractéristique additionnelle du dispositif selon l'invention, les moyens d'ajustement des paramètres du fonctionnement de la source de chaleur consistent en des moyens de sélection dans une mémoire raccordée à ladite unité centrale et le choix d'un profil déterminé parmi plusieurs profils préenregistrés dans la mémoire. En effet nous savons qu'une température optimale doit être obtenue pour avoir la meilleure réaction d'activation possible des principes actifs du peroxyde. En dessous ou au-dessus de cette température la molécule est moins active. La mémoire a pour but d'optimiser le temps d'action et la température obtenue par effet ohm naturel ou par la résistance électrique en fonction du degré d'action désiré. Ces paramètres issus de tests en laboratoire et bien connus des spécialistes nous permettent de prérégler à priori la valeur de la différence de potentiel entre les deux bornes de l'électrophorèse, donc, la puissance et le temps d'application en fonction du degré d'action désiré.

Selon une autre caractéristique additionnelle du dispositif selon l'invention, les moyens d'ajustement des paramètres du fonctionnement de l'électrophorèse et de la source thermique, sont préenregistrés dans la mémoire de l'unité centrale suivant certains paramètres, comme, le temps et la puissance et sont ajustables par le praticien.

Avantageusement dans un autre cas, un seul paramètre comme le temps est modifiable évitant toute fausse manipulation et ce en particulier dans le cadre d'une utilisation privée.

Avantageusement, la mémoire raccordée à la dite unité centrale peut être de type programmable pour l'enregistrement des profils thermiques et/ou des données relatives à un ou plusieurs paramètres ajustables comme le voltage ou l'intensité du courant, aptes à être sélectionnés.

Préférentiellement, le dispositif comporte des moyens de saisie tel qu'un clavier à touches et/ou un écran tactile et/ou tout autre moyen de saisie, notamment à distance, pour l'enregistrement dans la mémoire de profil énergétique et/ou de données correspondant à un paramètre ajustable de la mémoire. Dans ce dernier cas le praticien ou le patient pourra suivre l'évolution du traitement sur un écran, par Internet ou par modem confidentiel ou par tout autre moyen de transmission d'information.

Parmi les moyens de saisie à distance, figurent les paramètres de réglage des profils, le contrôle du fonctionnement de l'appareil et la recherche d'une panne éventuelle permettant ainsi sa télésurveillance, télédiagnostic ou télémaintenance sans l'intervention physique sur le site utilisateur.

Selon un dispositif additionnel de l'invention, la gouttière renferme des capteurs pour mesurer de courant ou des senseurs thermiques ou optiques permettant de connaître l'évolution du traitement avant de retirer la gouttière. En effet nous savons qu'il est impossible aujourd'hui de suivre et de connaître l'état réel de l'évolution du traitement de blanchiment avant le retrait de la gouttière. Selon un dispositif additionnel de la présente invention, cela consiste à proposer au praticien un moyen de connaître cette évolution donc de retirer la gouttière ou d'agir en feed back sur les paramètres comme la différence de potentiel, l'intensité ou le temps en suivant le comportement du courant car nous savons que plus une électrophorèse évolue et plus les caractéristiques du courant évoluent elles aussi. A priori nous pouvons tester la variation du courant électrique sur banc in vitro et la reporter in vivo afin de suivre à priori le blanchiment sans être obligé de retirer la gouttière.

Selon l'invention, il existe une alternative à l'activation thermique. Il s'agit d'une activation photonique pouvant être incluse dans la gouttière et comportant une source lumineuse et un acheminement de la lumière sur le site devant la recevoir. Ce mode d'action peut être une alternative obligatoire car nous savons que certains peroxydes comme ceux présents dans le "ultra light" commercialisé par "SHOFU" sont des produits plus photosensibles que thermos sensibles. Il existe donc une source lumineuse qui peut être halogène, par LEDs ou plasmatique comme l'Apollo 95^{E} ou toute association entre elles et dont la lumière est conduite par une fibre jusque dans la gouttière. Ce rayonnement assure, d'une part, une activation photonique correspondant à la longueur d'onde choisie en fonction du spectre d'absorption de la molécule de peroxyde et d'autre part, une activation thermique due au dégagement de chaleur accompagnant tout rayonnement halogène ou plasmatique.

Avantageusement, cette source lumineuse se compose d'une ou plusieurs diodes électroluminescentes capables (LEDs) d'émettre dans la zone photosensible des activateurs de la réaction de blanchiment des dents se situant entre 380 et 900 nanomètres. Ces diodes, réparties régulièrement sur la gouttière, par exemple en face de chaque dent, permettent à la lumière d'agir en une seule fois sur la totalité de l'arcade.

Avantageusement, ces diodes utilisent 2 sources de longueur d'onde, l'une activant la camphoroquinone ou la PPDA (470 et 430 nanomètres) et/ou une autre produisant un rayonnement thermique grâce à des LEDs émettant dans le rouge et l'infrarouge (au-dessus de 650 nanomètres). Les LEDs étant petites, elles peuvent être déposées régulièrement sur un porte-empreinte et alternativement pour bien répartir les longueurs d'onde sur l'arcade. Aucune fibre de conduction de lumière n'est nécessaire car les LEDs sont suffisamment petites pour être introduites dans la bouche du patient. Seul un cordon d'alimentation relie les LEDs à la source énergétique. Cette carte sera préférentiellement une alimentation DC-DC telle que celle décrite dans le Brevet FR 2.818.892. En effet le mode d'alimentation peut être le même qu'il s'agisse d'une réaction d'activation (peroxyde) ou de condensation (polymérisation).

Avantageusement, ces sources lumineuses ont un profil de type programmable variable en temps, longueur d'onde ou de puissance, pulsé ou non, permettant d'optimiser cette réaction d'activation.

Avantageusement et suivant ce mode d'action, il existera un découplage entre l'activation de la molécule de peroxyde, via la camphoroquinone, et la libération de l'ion fluor. En effet nous savons que l'ion fluor peut être inhibiteur de certaines réactions. Il est donc souhaitable qu'il soit libéré spécifiquement dans l'action électrophorétique après l'action du peroxyde, c'est-à-dire après la dégradation des molécules responsables de la coloration. Pour ce faire, l'activation photonique de la molécule de camphoroquinone et la libération du fluor se fera, suivant un mode alternatif de l'invention, par deux longueurs d'onde différentes, l'une émise par des LEDs à 470 nm pour la camphoroquinone, et l'autre par des LEDs dont le rayonnement sera différent et libérera la molécule de fluor à la fin de l'opération clinique de blanchiment.

En effet, et selon le corps de l'invention, il existe un dispositif permettant de combler les dégradations de la structure dentaire lors de l'évacuation des molécules de coloration.

Nous nous sommes rendu compte que les molécules de coloration pénétrant le plus profondément dans la dent, se logent, soit dans des espaces inter cristaux, soit dans le cristal lui-même lorsque celui-ci n'est pas complètement structuré à la suite de déficits alimentaires ou de traitements médicamenteux lors de sa formation (antibiothérapie à la tétracycline...NATHOO 1997). Cette situation d'une part, rend difficile son évacuation justifiant des concentrations élevées de peroxyde et d'autre part, et ceci est dangereux pour la dent, une dégradation du cristal lui-même justifiant la restriction de l'utilisation des produits de blanchiment ou la limitation dans le pourcentage des agents actifs comme le peroxyde d'hydrogène par les instances internationales de la protection de la santé. On sait depuis longtemps que l'extraction d'un ion, hydroxyle, calcium ou son absence, rend le cristal d'hydroxyapatite, instable et vulnérable. C'est souvent l'origine de la carie. C'est pour cela que nous recommandons des fluorisations de la dent afin de combler ces manques ioniques de fluor ou de calcium.

La présente invention, propose donc, d'utiliser la résistance électrique ou une résistance complémentaire comme champ de polarisation, permettant l'évacuation active des molécules colorantes et utilisées pour le blanchiment (peroxyde), mais aussi, la création d'un champ inverse, simultané ou à posteriori, permettant une action ionique réparatrice comme l'est la fluoration de l'organe dentaire.

En effet, nous savons que le fluor est l'un des éléments déterminant pour permettre de restructurer le cristal de la dent, émail ou dentine. C'est pour cette raison que cet ion est administré chez les enfants et les adultes en cas de décalcification. Cet ion, en effet, a pour fonction de venir combler les déficits calciques dans le cristal d'apatite, permettant une stabilisation de la structure moléculaire. C'est aussi pour cette raison qu'il est placé dans les kits de blanchiment comme par exemple, le "Opalescence F1" qui renferme 0,11% (soit 1,1 ppm) pour 5% de peroxyde de carbamide, mais qu'il doit aujourd'hui diffuser de manière passive, donc aléatoire.

Avantageusement, la présente invention propose d'injecter cet ion, où un ion similaire dans son action sur la protection de la dent, en créant un champ électrique inverse à celui utilisé pour l'évacuation des molécules responsables de la coloration dentaire ou de même polarité (positif pour attirer le fluor, négatif, à l'intérieur de la dent) mais durant l'évacuation et après l'action du peroxyde. L'action peut se faire en même temps que l'action du peroxyde dans la mesure ou l'ion utilisé n'est pas inhibiteur. La présente invention propose de séquencer les polarités en fonction de l'effet désiré : action du peroxyde, évacuation des colorants et renforcement de la dent en fonction des inter-relations possibles des molécules les unes avec les autres. Le fait d'utiliser un mode électrophorétique, non seulement permet de déplacer ces molécules de manière active, mais garantit aussi de séparer les actions chimiques afin d'éviter toute action interactive pouvant être négative entre les différentes réactions. C'est la seule méthode permettant d'affirmer qu'il n'y a pas d'inter-relation chimique dans des phases devant êtres séparées.

Pour ce faire, nous pouvons résumer la présente invention comme étant un dispositif électrophorétique et chimique, accessoirement thermique, de blanchiment, comportant une source énergétique délivrant un courant électrophorétique variable dans sa polarité, et dont les fils conducteurs sont reliés, d'une part, au corps du patient (premier pôle), et d'autre part (deuxième pôle), à une gouttière renfermant les produits chimiques responsables d'un traitement de blanchiment. Ces fils conducteurs électriques présents dans la gouttière permettent à la fois l'élévation thermique par effet Joule (pouvant être remplacé par une source photonique) mais aussi, de par leur polarisation électrophorétique variable, le mouvement des molécules vers l'intérieur de la dent pour qu'elles soient rapidement et profondément actives (comme les molécules de peroxyde ou les ions fluor, molécules reconstitutives de la dent après le blanchiment), et le mouvement inverse vers l'extérieur de la dent assurant une évacuation active des molécules responsables de la coloration. Le champ électrophorétique se situe au niveau de la dent. L'un des pôles est tenu dans les mains ou sur le corps du patient, cette polarité est transmise via les voix sanguines et lymphatiques à la pulpe dentaire, et l'autre pôle est transmis par la résistance électrique présente dans la gouttière.

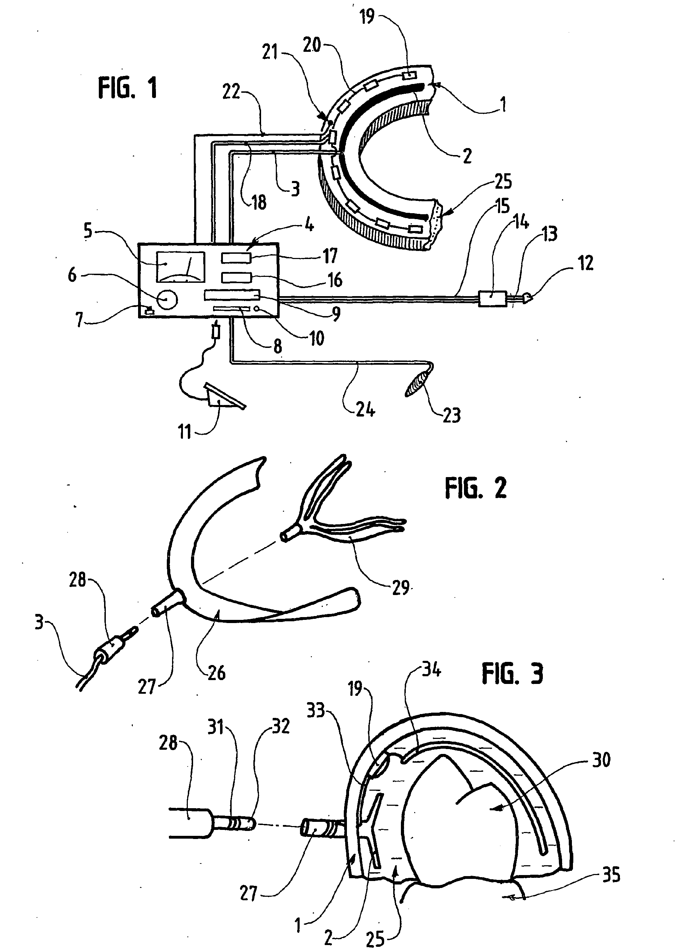

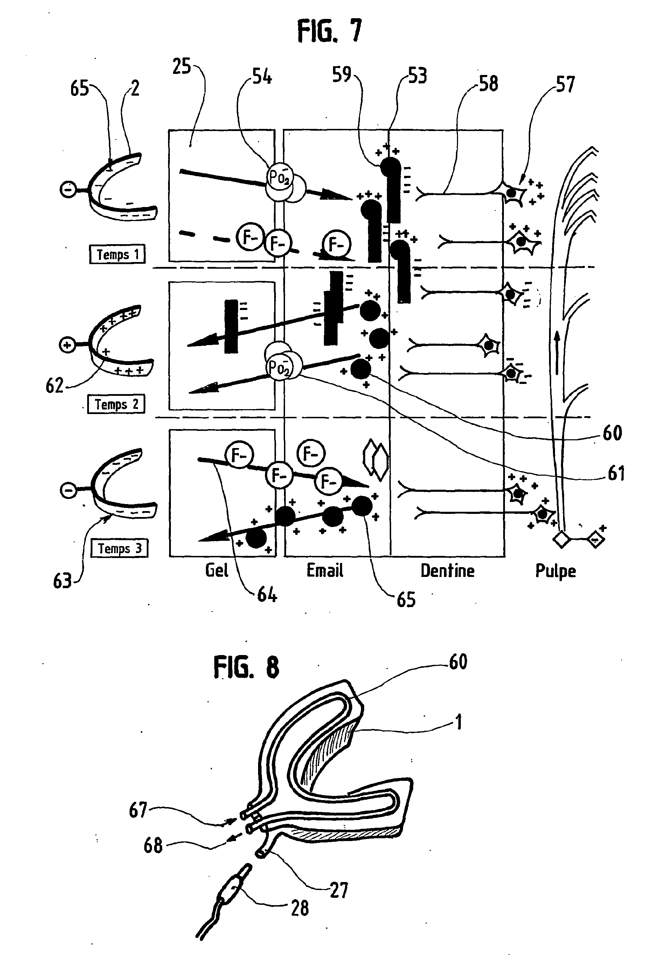

D'autres buts et avantages de la présente invention, apparaîtront dans la description qui va suivre, se rapportant à un mode de réalisation, donné à titre d'exemple indicatif et non limitatif. La compréhension de cette description sera facilitée au vu des dessins joints en annexe et dans lesquels :
- la figure 1 représente une vue schématique d'un dispositif pour le blanchiment des dents selon l'invention.
- la figure 2 représente une vue schématique partielle et en éclaté d'une partie du même dispositif.
- la figure 3 représente une vue schématique en coupe transversale d'une partie du même dispositif lors d'une opération de blanchiment.
- la figure 4 représente une vue schématique en coupe partielle d'une autre partie du même dispositif.
- les figures 5a et 5b sont des schémas de la représentation d'un cycle d'analyse de la couleur d'une dent.
- les figures 6a et 6 b et présentent des vues schématiques partielles de variante du même dispositif.
- la figure 7 est une illustration schématisée des différentes étapes du traitement mis en oeuvre par le dispositif selon l'invention.
- la figure 8 représente une vue schématique en perspective d'une variante d'une partie du même dispositif.
- la figure 9 représente un diagramme illustrant les différentes étapes du traitement mis en oeuvre par le dispositif selon l'invention.
- la figure 10 représente une vue schématique en coupe verticale d'une dent dévitalisée lors de son traitement avec le dispositif selon l'invention.
- la figure 11 représente une vue schématique partielle d'un mode de réalisation particulier du dispositif selon l'invention, adapté au traitement des ongles.
   Tel que représentée dans ces figures, la présente invention, est relative à un dispositif de blanchiment qui trouvera un intérêt tout particulier dans le domaine dentaire.
   Comme visible sur la figure 1, ce dispositif comporte une gouttière standard 1 avec sa résistance électrophorétique 2 et une rangée de LEDs activatrices 19 reliées par des connexions électriques 3 à un boîtier électronique de commande, de contrôle et de mémorisation 4 et à une poignée de polarisation inverse 23.
   La gouttière standard 1, destinée à recouvrir l'arcade dentaire à blanchir, est munie d'un système de conduction électrique 2 permettant la création d'un champ électrophorétique, accompagné d'une élévation thermique, ayant une polarité positive ou négative, et qui est relié, par exemple par un fil électrique 3, à une unité de réglage et de contrôle 4, contenue dans un boîtier, permettant de définir ou de programmer les paramètres du traitement clinique (temps, degré désiré par le patient...) et ceux définissant le champ électrophorétique (intensité, voltage...). Ces critères contrôlables sur des cadrans 5 peuvent être modifiés par manipulation de boutons de commande 6, télétransmis à distance dans un système géographiquement déporté et grâce à une liaison numérique (modem...) 7, mémorisés sur une disquette 8 ou stockés dans une unité centrale 9. Sur une prise 10 peut être installée une pédale 11 de déclenchement/arrêt de tout facteur intervenant comme la lumière d'activation et/ou du champ électrophorétique, et/ou d'un flash d'analyse spectrocolorimètre. La tête 12 de ce dernier reçoit, grâce à un conducteur 13, la lumière émise par des LEDs de référence qui peuvent être Rouge, Verte, Bleue, et par retour, l'information modifiée par l'objet mesuré, dans cet exemple une'dent. Cette conduction se fait par fibres optiques ou directement sur un capteur 14 qui envoie les valeurs mesurées à l'analyseur situé dans l'unité 4. La mise en route sélective du spectrocolorimètre se fait, via un moyen de conduction 15, grâce à un bouton 16 situé en façade de l'unité 4 et le résultat obtenu est visible sur un écran alpha-numérique 17 situé au-dessus. La mise en route du système calorique, grâce au bouton sélectif 16, conduit l'envoie d'énergie par un fil 18 qui induit soit l'éclairement de LEDs 19 situées sur la gouttière 1, soit l'élévation thermique d'une résistance 20 située dans la gouttière 1 si le fil créant le champ électrophorétique 2 est insuffisant. Une sonde 21, thermique ou électrique indiquera par retour l'état de l'électrophorèse à l'unité centrale de contrôle et d'information 4. La polarité inverse à celle créée par la résistance 2 sera transmise vers la pulpe, via le sang ou le système lymphatique, etc..., grâce à une poignée 23 tenue dans la main et reliée à l'unité 4 par un fil 24. Cette polarité peut-être inversée automatiquement ou par action sur le bouton 6.
   L'activation des peroxydes peut se faire grâce à l'élévation en température d'une résistance thermique commune avec celle servant a l'électrophorèse 2 ou indépendante de cette dernière 20 si le choix d'une forme et/ou d'un matériau différent peut optimiser l'une ou l'autre des fonctions.
   On observera tout particulièrement que la présente invention n'est nullement limitée à une activation électrique des composants basiques présents dans un gel de blanchiment 25 en composants actifs sur les produits colorants et sensibles à l'électrophorèse. En effet, cette activation peut se faire, selon l'invention, par des moyens optoélectroniques. Dans ce cas, une résistance 2 assure l'électrophorèse, et une source de lumière activatrice est conduite d'une lampe située dans l'unité électronique 4 jusqu'au gel de blanchiment par une fibre optique 18, ou produite directement par des LEDs à proximité de ce dernier 19. Dans ce dernier cas la fibre optique 18 est remplacée par un fil de conduction électrique qui assure l'alimentation des LEDs.
   Afin de contrôler par feed-back la température, les caractéristiques du courant électrophorétique et/ou la puissance lumineuse, il est prévu un capteur thermique, électrique et/ou photonique 21 relié à l'unité centrale de contrôle 4 par un câble de connexion 22, ou par une communication par ondes. Ces capteurs 21 envoient l'information nécessaire à l'unité centrale pour qu'elle contrôle si le traitement se fait dans le respect des réglages programmés dans l'unité centrale 4. Ce retour d'information permet le réglage régulier voir continu et automatique dans le respect des fonctions programmées. Il va de soi, bien sur, que l'unité centrale n'est pas obligatoire pour la mise en oeuvre de l'invention, et qu'un simple réglage manuel peut donner satisfaction dans une utilisation rustique.
   Si nous décidons d'utiliser un gel 25 sensible au courant électrophorétique produit par les pôles 2 et 23, et qui a pour caractéristique de changer de couleur au moment de son activation puis de sa migration, ce capteur 21 peut être une cellule photosensible, comme une photodiode. Dans ce cas, en appliquant la loi de Bert Lambert, qui relie les concentrations aux intensités mesurées par la photodiode suivant la formule Ic / Io = log Cc / log Co, nous pourrons suivre l'évolution de la concentration des principes actifs dans la dent et le retour des molécules colorées dégradées. La projection de la lumière d'analyse se fait au niveau de la tête 12 du colorimètre alors que la lecture est enregistrée grâce à un CCD ou une photodiode située dans la tête 12 ou dans le capteur 14.
   En référence maintenant à la figure 2, qui est une représentation schématisée d'une plaque de conduction souple pouvant être plaquée à la surface des dents avant que soit positionnée une gouttière adaptée à la bouche du patient de manière traditionnelle, nous voyons que la plaque de conduction électrophorétique 2 peut être constituée d'un conducteur souple 26 pouvant être plaqué à la surface des dents avant que soit positionnée une gouttière spécialement moulée et adaptée à la bouche du patient de manière traditionnelle par le dentiste ou le prothésiste. Dans ce cas la plaque souple 26 est plaquée sur les dents ou pré-moulée sur l'empreinte du patient. Suivant un travail parfaitement connu des professionnels, il est ensuite réalisé une gouttière anatomique, c'est-à-dire spécifiquement moulée en résine sur la forme de la bouche du patient. Cette gouttière peut être réalisée dans un matériau transparent pour permettre l'activation du gel peroxydé (ou actif par un autre principe chimique) par une lumière appliquée de l'extérieur. Dans ce cas le filament électrophorétique devra le plus possible être plaqué sur la dent ou devra être constitué d'un matériau conducteur lui-même transparent comme des résines chargées. L'apport électrique se fait par l'intermédiaire d'un connecteur 27 et de son complémentaire 28 si l'on souhaite pouvoir séparer la gouttière 1 de l'unité 4 pour des raisons pratiques évidentes.
   En référence à la figure 3, qui représente une section de la gouttière 1 on peut voir que la prise 28 est divisée en deux parties, l'une pour l'alimentation 31 et l'autre pour la commande on/off de la résistance thermique par énergie thermique 34 ou photonique si l'on utilise les LEDs 19 ou toute autre source de lumière. Le gel 25 étant conducteur, la résistance 2 de création de champ électrophorétique n'a pas besoin d'être trop importante, la polarisation du champ étant parfaitement dispersée. La lumière 19 ou la résistance thermique 34 a pour fonction de favoriser le déclenchement de la réaction d'activation du gel de blanchiment 25 se trouvant au contact de la dent 30 et un peu de la gencive 35 même si cela n'est pas souhaitable du fait de l'agressivité naturelle des peroxydes sur les tissus mous.
   On notera que, de manière non limitative, la prise 28 est de type jack. Elle peut comporter plus d'un contact séparé s'il s'agit de joindre le contrôle par feed-back ou plusieurs autres types de polarisation.

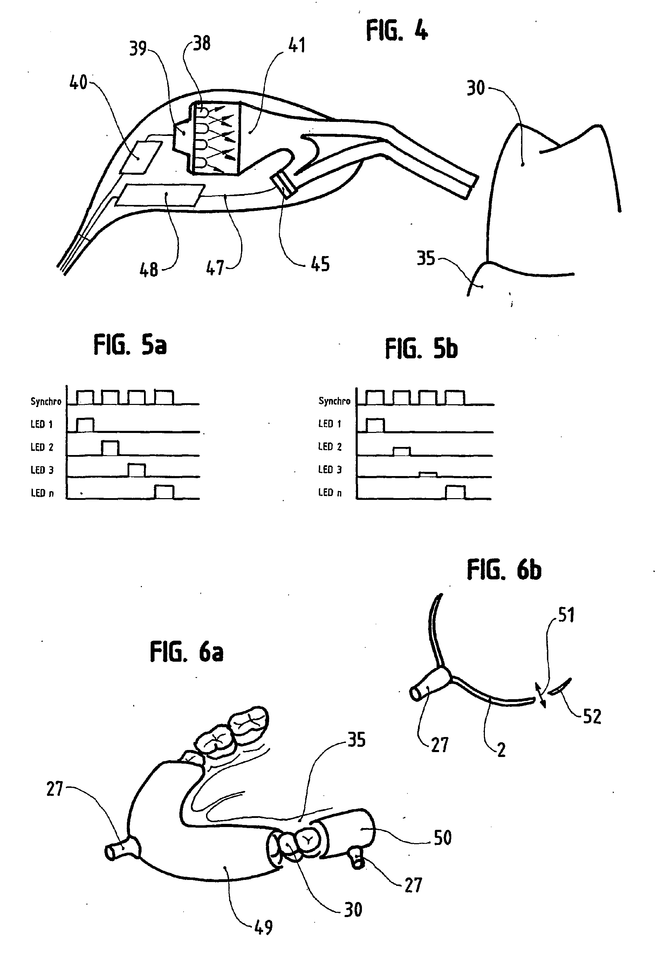

   Sur la figure 4 est représenté un colorimètre différentiel 14 peu coûteux et servant d'unité de contrôle de l'efficacité du traitement de blanchiment par électrophorèse, tandis que les figures 5a et 5b sont des schémas, respectivement A et B, de la représentation d'un cycle d'analyse de la couleur d'une dent, respectivement avant et après blanchiment. Le colorimètre différentiel est un système très simple basé sur la mesure différentielle d'une couleur, avant et après traitement par réflexion sur la dent 30.
   Le principe de la manipulation est le suivant: on étalonne une source lumineuse composée de plusieurs LEDs 38 fixées sur un support évacuant le dégagement de chaleur 39, alimentée et commandée par une carte DC/DC 40. Ces LEDs sont en nombre (minimum de 2) et de couleur variable, mais de préférence Rouge, Verte et Bleue (système RGB). Elles émettent un rayonnement qui est repris par un système concentrateur optique 41 puis conduits, après séparation dans un espace pour partie sur la dent 30 et pour partie directement sur un senseur 45 (en général une photo-diode, un CCD ou un Cmos), avec ou sans mixage. Il est possible de séparer un même faisceau en deux parties et de faire la mesure sur deux ou un seul capteur. Il est possible aussi de n'utiliser qu'un seul senseur 45 en décalant la mesure dans le temps. Les informations seront conduites à partir du senseur 45 via une liaison 47, jusqu'à un comparateur différentiel 48 qui, grâce à l'analyse de la différence entre le faisceau incident, schéma A, et le faisceau réfléchi, schéma B, pourra donner une valeur de la teinte de la dent et pourra la comparer avec la valeur mesurée avant traitement.
   Si l'on a pris soin de mémoriser dans l'unité centrale 9 les teintes dentaires standards bien connues par l'homme de l'art (teintes Vita pour les dents...), avec la même méthode, et la même référence (test étalon), il est possible de voir, sur l'écran alpha-numérique 17, et de suivre l'évolution, donc, l'efficacité du traitement en fonction de critères reconnus par les professionnels. Il est donc possible selon l'invention de faire une mesure objective des résultats du traitement sans être obligé d'avoir recours à des méthodes trop coûteuses.
   Comme l'indique la figure 6a, la gouttière peut n'être que partielle 49 ou mono dentaire 50 si l'on ne souhaite pas traiter certaines dents 30 pour des questions médicales (douleurs aux collets ...) ou si l'on a des gencives 35 sensibles. Le connecteur 27 sera de préférence identique quel que soit le type de gouttière.
   De même, et comme cela est représenté sur la figure 6b, il est possible de disposer d'une connexion 27, sans la gouttière, reliée simplement au fil d'électrophorèse 2 dont la longueur peut être adaptée, par la réalisation d'une section 51 pour éliminer une partie 52, afin de le limiter à quelques dents.
   Afin que soit bien comprise la fonction du dispositif, objet de la présente invention, a été représentée sur la figure 7, l'action des champs électriques électrophorétiques tel que mise en oeuvre. Nous noterons à titre d'exemple qu'il peut exister, et ceci n'est pas limitatif, trois temps successifs d'action utilisant les courants électrophorétiques de blanchiment.
- Dans un premier temps le courant électrophorétique entraîne vers l'intérieur de la dent 53 les molécules riches en produit de blanchiment, comme par exemple le peroxyde de carbamide 54, présent dans le gel 25. Si les molécules porteuses des groupements actifs sont chargées négativement, la polarité 55 de la résistance de la gouttière est négative et la polarité du corps, tenu par exemple dans les mains du patient est positive. Cette polarité est transmise dans toute la dent, via les cellules odontoblastiques bordeuses de la pulpe 58, principalement à l'interface 53 dentine / émail où se trouve souvent une concentration de molécules responsables de la coloration 59. Nous aurons donc un mouvement ionique vers le centre de la dent, poussant les molécules actives contre ces colorants. Si la molécule active n'est pas chargée, on peut l'entraîner passivement dans les mouvements ioniques créés par des fluides additionnels composés de molécules très chargées.
- Dans un deuxième temps, est créé un mouvement inverse et unique, coeur de l'invention, et ayant pour but d'extraire de la dent les molécules colorantes 60 ayant été dégradées par les agents de blanchiment 61. Il va de soi que se sont les molécules colorantes d'une polarité inverse de celle de la résistance qui seront extraites de la dent. Cette polarité présente dans le gel 62, étant la même que celle des molécules de blanchiment 54, les molécules de blanchiment non dégradées ou qui n'ont pas réagit, sont extraites de la dent pour éviter qu'elles poursuivent leur action au-delà du traitement et qu'elles risquent de léser les composants de la dent (ce qui les rend hypersensibles et peut même les dégrader). Cela permet aussi de stopper réellement le procédé à un moment défini et choisi. Cette technique est la seule qui rend contrôlable le blanchiment des dents.
- Enfin dans un troisième temps, mais ceci n'est pas limitatif dans le principe de l'invention, le gel résultant de l'action des deux premiers temps, chargé des colorants et des molécules du produit actif ayant réagi, ou non, comme les peroxydes, est retiré et remplacé par un gel riche en fluor, ou/et en tout autre produit reconstitutif de la dent, chargé électriquement comme la molécule porteuse des groupements actifs (peroxyde). La polarité de la résistance présente dans le gel est à nouveau inversée 63 afin de permettre, d'une part, la pénétration des ions fluors (ou autres) 64 et d'autre part d'attirer les molécules de colorant ayant une polarité inverse et ayant été retenues dans la dent lors du temps 2.

Il va de soi que, et ceci est aussi dans le principe de l'invention, le fluor peut être présent dans le produit actif de blanchiment et migrer avec le gel dans le temps 1. Les polarités sont compatibles. Cette solution a l'avantage d'éviter le changement du gel au temps 3 car il suffit alors d'inverser deux fois les polarités. Par contre ceci a l'inconvénient de faire côtoyer deux molécules qui peuvent être incompatibles, le fluor étant un inhibiteur de réaction bien connu.

Avantageusement, si l'on souhaite accélérer la procédure clinique, on peut ne procéder qu'à une seule polarisation dans le sens de la pénétration des molécules actives, avec ou sans fluor ou de la sortie des molécules dégradées après action des molécules actives hors champ électrique. Cette méthode peut être intéressante si le comportement de certaines molécules actives est troublé durant leurs réactions sur les colorants.

Il peut aussi être programmé, selon l'invention, des séquences sans courant électrophorétique, par exemple, entre les temps 1 et 2 et entre les temps 2 et 3. Ceci peut permettre de parfaire certaines réactions sensibles au courant électrique.

De même, toute forme d'intervention sur le courant électrophorétique est envisageable dans le cadre de l'invention. Ainsi on peut, de manière non limitative :
- Jouer sur l'intensité. Nous avons démontré expérimentalement qu'il est possible d'augmenter significativement l'action de la technique selon l'invention en faisant croître puis décroître rapidement l'intensité du courant, et ceci, par vagues successives.
- Faire varier la polarisation des pôles, non pas en trois temps, mais de manière alternative, pour permettre à certaines molécules de trouver des chemins plus favorables à leur trajectoire dans le cristal de la dent.
- Utiliser ce principe alternatif, en jouant sur le temps accordé à chaque polarisation. Nous gardons les temps tels que définis précédemment, mais nous inversons plusieurs fois et rapidement la polarité. Nous avons globalement une polarisation positive ou négative correspondant aux temps évoqués mais elle est entrecoupée de polarité inverse permettant aux molécules de circuler dans la dent, et éventuellement, de se dégager d'impasses stériques ou électriques.

Sur la figure 8, selon une variante, l'élévation thermique est apportée, non pas par une résistance électrique, mais par une circulation d'eau ou de tout type de vecteur thermique dans des conduits 66 qui parcourent la gouttière 1 en pénétrant par un orifice 67 et ressortant par un autre 67. Ce vecteur peut être un liquide ou un gaz comme de l'air chaud. Il va de soi que ce montage, n'empêche pas d'adjonction du champ électrophorétique. Dans l'exemple représenté sur cette figure, et correspondant à une possibilité de la mise en oeuvre de l'invention, le champ est porté par la gouttière 1 elle-même, qui est en matériau conducteur, et la connexion 27-28 est visible sous les conduits du vecteur thermique.

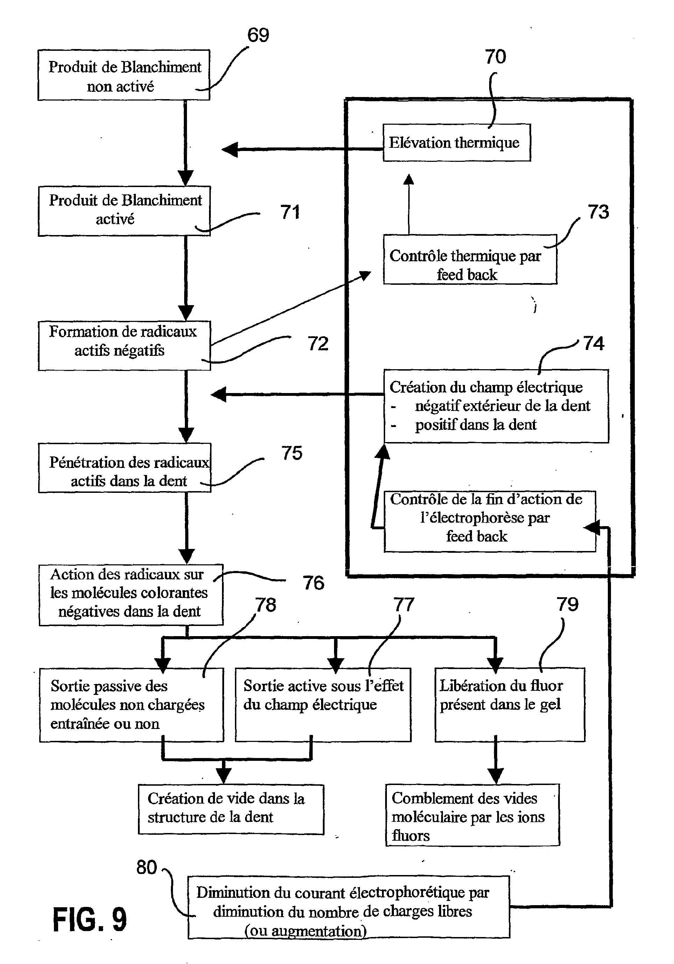

Le diagramme de la figure 9 illustre les étapes successives de fonctionnement, dans le cas ou les molécules responsables de la coloration de la dent sont de charge positive. Le produit non activé est placé dans une gouttière 69 standard ou réalisée sur mesure. Le principe d'activation ou d'accélération de l'activation de la molécule à base de peroxyde ou autre est mis en route grâce à une élévation thermique ou photonique 70. Le produit activé 71 présente une valence négative (dans notre exemple) 72. Un contrôle par feed-back peut limiter à tout moment l'apport énergétique, en mesurant directement la température 73 ou/et en suivant l'évolution du courant électrophorétique. C'est en effet à cet instant que l'on crée le champ électrophorétique en mettant le contact sur la gouttière (négatif), et en demandant au patient de tenir l'autre pôle (positif). 74. Il y a pénétration des radicaux négatifs actifs dans la dent 75, attirés par les charges positives de la pulpe et repoussés par les charges négatives de la gouttière. Les molécules de peroxyde sont alors en contact direct avec les molécules de colorant 76. Ceci permet de diminuer significativement les doses actuellement utilisées, donc, de protéger la dent. Nous inversons ensuite la polarité. Les molécules de colorant dégradées et chargées positivement sortent, attirées, par le pôle négatif de la gouttière 77 accompagnées des molécules de peroxyde et des molécules non chargées pouvant être entraînées passivement 78. Nous inversons une dernière fois la polarité afin de permettre la pénétration du fluor 79. Le feed-back mesurant les caractéristiques physiques du courant électrophorétique peut intervenir à tous niveaux 80.

Avantageusement et selon l'invention, les molécules des produits actifs, comme le peroxyde de carbamide, sont chargées lors de leur conditionnement, évitant ainsi la phase d'activation, où deviennent chargées lors de la phase d'activation par des moyens énergétiques décrits précédemment. Ces charges peuvent être portées par un radical de la molécule indépendant du radical de peroxyde ou de tout autre produit blanchissant (réducteur et/ou oxydant).

Il est rappelé aussi que selon l'invention, et pour favoriser les mouvements des molécules non chargées intervenant dans l'acte de blanchiment, il est possible de mouvoir, grâce à une action électrophorétique indirecte, les molécules, en les entraînant par un fluide chargé présent dans le gel ou ajouté à ce dernier. A cet effet, et selon l'invention, est prévu un gel capable de pénétrer dans la dent, sans action chimique sur les molécules des colorants, mais très dynamique. L'inversion de polarité dans la dent entraînera son retour à l'extérieur de la dent, entraînant par ce second mouvement inversé (dynamisme des fluides) les molécules dégradées et colorantes.

Avantageusement le gel renfermant le produit blanchissant (par exemple le peroxyde de carbamide) et/ou le produit renforçateur de la dent (par exemple le fluor) change de couleur en fonction de la concentration. Par exemple le mouvement des groupements peroxyde dans la dent diminue sa concentration dans le gel en l'augmentant dans la dent. Ceci permet, selon l'invention, de suivre la progression de la procédure clinique, et de l'arrêter à une couleur connue comme correspondant à une bonne concentration des groupements actifs du peroxyde dans la dent, et d'assurer une bonne action de blanchiment. La même observation peut être faite lors de la migration du fluor (par exemple) ou le retour des colorants.

Avantageusement et selon l'invention, le gel renferme des molécules qui, en contact avec les colorants, deviennent d'une charge inverse de celle présente dans la molécule de blanchiment (peroxyde) ou transforment la charge du colorant dégradé en charge inverse du peroxyde. Cette méthode a l'avantage de supprimer le temps 2 de l'électrophorèse, car les charges des colorants et celles des colorants dégradés, sont dans ce cas, et selon l'invention, inverse des molécules actives de blanchiment (peroxyde). Elles subiront un mouvement de sortie de la dent opposé et simultané à celui de pénétration du peroxyde sans que l'on ait besoin d'inverser la polarité. Il y a donc fusion des temps 1 et 2, décrits dans l'exemple de la figure 7 et seulement deux temps d'action: les temps 1 et 3. Si l'agent renforçateur de la dent (fluor), est présent dans le gel initial, il n'est plus nécessaire de créer une inversion de polarité, et un seul temps est alors possible.

Il va de soi que selon l'invention, les produits chargés étant très vulnérables, l'activation et la polarisation des composés actifs comme le peroxyde de carbamide se fait par mélange des composés de base, avant leur introduction dans la gouttière.

Pour répondre à un souci d'automatisation, nous pouvons adjoindre dans le gel électrophorétique un capteur 21 mesureur de courant (différence de potentiel, intensité...). En effet, nous savons que durant l'électrophorèse il y a variation progressive des caractéristiques du courant mis en jeu, en particulier lorsque le mouvement ionique est terminé, ces valeurs tendent vers l'infini, il est donc facile de prévoir un arrêt automatique, ou une indication de fin de cycle, donc de savoir si le traitement est terminé sur des critères scientifiques pré mémorisés.

Il va de soi aussi que, selon l'invention, la résistance thermique/électrique de l'électrophorèse servira pour le contrôle par feed-back de l'émission lumineuse. Les valeurs enregistrées permettent de savoir s'il faut, ou non, arrêter l'action photonique de la lumière. Il est également possible de faire varier cette puissance lumineuse en ajustant les paramètres contrôlant la puissance électrique alimentant les LEDs. La mise en jeu du procédé DC-DC tel que décrit dans le document WO 03/068102 permet d'obtenir ce résultat dans les meilleures conditions.

A ce concept de contrôle de l'énergie mise en oeuvre dans le blanchiment peuvent être associés d'autres systèmes utilisés couramment chez le praticien ou au domicile du patient. Ainsi et selon l'invention, l'alimentation utilisée à domicile pour l'électrophorèse, peut être partagée avec celle utilisée pour l'alimentation ou la recharge des brosses à dent électrique ou des téléphones portables (en voyage). Cette dualité permet de réduire significativement le prix d'un tel dispositif. De même chez le dentiste, l'association de ce système électrophorétique à des ensembles ultrasoniques ou à des projections de produits abrasifs (bicarbonate...) permet de concevoir un centre de blanchiment incorporant l'ensemble des appareils utilisés par l'homme de l'art, sans en augmenter significativement le coût, puisque les éléments les plus coûteux (alimentation ...), sont mis en commun pour plusieurs technologies.

Avantageusement le réglage et le contrôle des paramètres permettant la mise en oeuvre de cette invention est sous la dépendance d'un CPU, lui-même, programmé par l'homme de l'art. Il existe un système de carte à puce permettant de programmer ces paramètres, et il suffit au patient d'introduire la carte que lui a remise son dentiste pour être sur que les paramètres définissant l'électrophorèse sont conformes aux règles de l'art. En fin de compte, le dispositif conforme à l'invention, donne la possibilité à l'utilisateur de régler et d'ajuster les conditions de fonctionnement de son appareil comme et où il le souhaite, de sorte qu'il n'est pas limité comme par le passé à l'action aveugle et empirique du peroxyde sans contrôle ni suivi.

Avantageusement et selon l'invention, le colorimètre 14 est associé à ce contrôle par feed -back. Nous savons que la couleur d'un corps est fonction de 3 critères dit L.A.B. , xy et Y, RGB ou autres, suivant la théorie suivie. Le fait d'utiliser dans le dispositif un détecteur de couleur 14 permet de suivre l'évolution du blanchiment en cours de traitement. Cette mesure peut se faire, selon l'invention, au niveau du gel qui peut se décolorer ou se colorer suivant l'apport des molécules colorantes ou par réaction avec ces dernières, comme nous l'avons décrit précédemment. Cela permet de savoir si l'on doit ou non arrêter l'application, et évite d'avoir à procéder de façon empirique. Cela évite aussi d'arrêter l'action avant la fin du traitement et permet de limiter les frais engagés. De même, ce système de mesure peut se faire sur la dent, avant et après traitement, permettant ainsi au praticien de montrer à son patient l'évolution du blanchiment. Pour ce faire un capteur spécial 12 composé du mesureur de couleur décrit précisément dans la figure 4, a été développé et dont l'extrémité peut :
- soit s'intégrer au gel,
- soit se poser sur la dent.

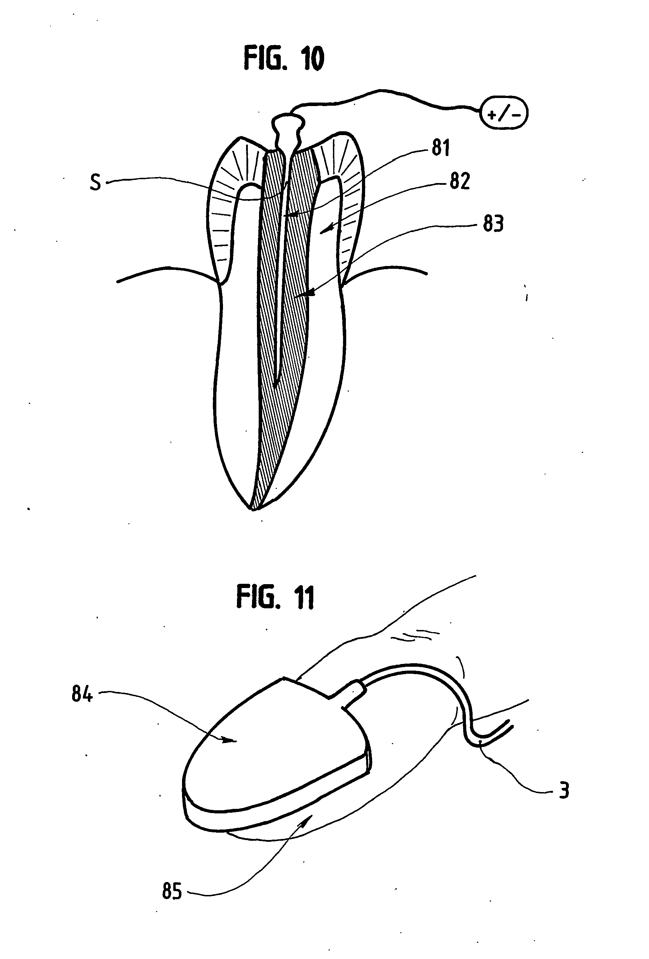

Il n'en reste pas moins vrai, que la plupart des dispositifs de blanchiment ne peuvent pas agir sur des dents dévitalisées ou le font moyennant de fortes concentrations ou avec l'utilisation de produits spécifiques comme les borates. Il en serait de même de la présente invention si une solution n'était pas proposée puisque, la pulpe et la circulation sanguine ayant été retirées, il ne peut plus y avoir conduction électrique de la polarité inverse à celle du gel, à l'intérieur de la dent. La présente invention propose une alternative spécialement développée pour les dents dévitalisées. Avantageusement et selon l'invention, comme on le voit figure 10, le pôle tenu dans la main du patient ou placé contre son corps, est remplacé ou est couplé (en série) avec une sonde S que l'on peut introduire dans le canal dentaire 81 de la dent dévitalisée 82 et préalablement alésée. Un gel conducteur 83 est placé dans le canal en remplacement de la pulpe dentaire qui n'existe plus, après que l'on ait pris soins d'humidifier la dent. La fonction de conduction du deuxième pôle est donc ainsi récréé même en absence de pulpe.

Avantageusement et toujours selon l'invention, ce gel renferme des composants de blanchiment actif et du fluor comme nous l'avons observé dans le gel externe, mais de polarité inverse, de telle sorte qu'ils soient attirés vers le gel de cette gouttière externe. Nous créons un deuxième mouvement de molécules actives inverse à celui de la gouttière du pôle externe. S'il n'existe pas de molécules actives de polarité inverse, il suffit de répéter l'opération de manière séquentielle: blanchiment externe, puis, blanchiment interne etc ... en utilisant le même gel et la même polarité (tel que décrit dans la figure 7, mais en ayant une inversion de polarité, suivant que le gel actif se trouve à l'intérieur ou à l'extérieur de la dent.

Cette extension de l'invention a l'avantage de permettre une technique de blanchiment des dents dévitalisées, ce qui est très important car ce sont souvent les dents les plus colorées. Cela a aussi l'avantage d'augmenter encore plus significativement l'action des produits par le fait qu'ils se sont déplacés électrophorétiquement vers les molécules colorées de l'extérieur et de l'intérieur de la dent. A cette double action interne et externe est associée une action d'extirpation des molécules de colorant des deux cotés. Dans ce cas, et pour comprendre le mécanisme de blanchiment depuis le pôle interne, il suffit d'inverser les temps 2 et 3 de la figure 7.

En référence maintenant à la figure 11, on peut voir que l'invention ne se limite pas au blanchiment des dents, mais qu'elle trouve également une application au traitement d'autres parties du corps comme des tissus ou des poils, et en l'occurrence les ongles. Cette figure montre ainsi un moyen 84 support de gel, adapté à l'extrémité d'un doigt 85, et recouvrant l'ongle, non visible, de ce dernier.

Il ressort de la description qui précède que la présente invention, répond parfaitement aux problèmes posés, en ce sens qu'elle apporte une réelle réponse pour l'optimisation du blanchiment des dents, et la protection de l'organe dentaire. Il ressort aussi de cette description, qu'elle permet, de résoudre des problèmes fondamentaux, comme le contrôle de l'acte clinique, ce qu'aucune autre méthode n'a encore proposé.

Il va de soi que l'invention ne se limite pas au seul mode de mise en oeuvre de ce procédé, ni aux seules formes d'exécution du dispositif pour la mise en oeuvre de ce procédé, décrites ci-dessus à titres d'exemples ; elle en embrasse, au contraire, toutes les variantes de mise en oeuvre et de réalisation. C'est ainsi, notamment, qu'il serait possible de blanchir d'autres objets que des parties du corps humain, comme, par exemple, des statues ou des tissus. Il est aussi possible de prévoir un type de blanchissage industriel basé sur la présente invention et assurant les mouvements de pénétration du corps nettoyant ou d'extirpation des molécules responsables de la coloration par électrophorèse.

## Revendications

1. Dispositif pour le blanchiment utilisant des moyens électro-optiques et chimiques, notamment dans le domaine médical et dentaire en vue de l'éclaircissement de parties du corps comme les dents, les poils ou les ongles, **caractérisé en ce qu'**il comprend :
- un générateur/contrôleur (4) de courant d'onde électrique, électrophorétique ou électromagnétique de profil et/ou de modulation variable,
- des moyens (3) pour transmettre ce courant vers lesdites parties à éclaircir,
- des moyens pour créer un champ électrique, électrophorétique ou électromagnétique non traumatisant (2, 26, 29, 49) au travers de la zone traitée, comprenant des électrodes et un gel conducteur (25),
- des moyens (1, 84) pour contenir ledit gel (25) au contact des pôles et du corps permettant la continuité de ce champ,
- des produits actifs (25) mis en jeu pour le blanchiment, sensibles aux courants électrophorétiques,
- des moyens pour activer lesdits produits soit par la lumière (19), soit par la chaleur (27).

2. Dispositif selon la revendication 1, **caractérisé en ce qu'**il comporte une unité centrale (8, 9, 10) de gestion, de mémorisation et de contrôle direct par l'opérateur où indirect par mémoire ou par Internet des paramètres de fonctionnement de la source électrique, électrophorétique où électromagnétique afin de permettre de maîtriser et de simplifier la pénétration de toutes ou parties des molécules sensibles au champ électrique, électrophorétique où électromagnétique, actives pour le blanchiment et la protection de la partie cible du traitement et la sortie de tout ou partie des molécules sensibles au champ électrique, électrophorétique ou électromagnétique et indésirables ou ayant réagi dans ce processus d'éclaircissement et pouvant être retirées du corps mais aussi des moyens de contrôle directs par feed-back sur l'unité centrale, ou manuel par l'opérateur des paramètres permettant de connaître l'évolution et les résultats de l'acte de blanchiment pratiqué, comme le sont les variations thermiques ou électriques (21) et la modification de la coloration (12) induites par le traitement.

3. Dispositif selon la revendication 1 ou la revendication 2, **caractérisé en ce qu'**il comporte un générateur/contrôleur (4) de courant électrophorétique, un circuit de conduction (3) et une résistance créant un pôle dans les moyens (1, 84) pour contenir le gel (25), le deuxième pôle étant en contact d'une partie de corps.

4. Dispositif selon l'une quelconque des revendications précédentes, **caractérisé en ce que** les moyens (1, 84) pour contenir le gel sont modelés (49) sur la partie du corps du patient.

5. Dispositif selon l'une quelconque des revendications 1 à 3, **caractérisé en ce que** le deuxième pôle situé en contact du corps est adapté pour se placer dans un tube contenant un gel électrophorétique de blanchiment comme la racine (81) d'une dent dévitalisée (82).

6. Dispositif selon l'une quelconque des revendications précédentes, **caractérisé en ce qu'**il comporte un activateur photonique (19) apte à activer les produits (25) utilisés pour le blanchiment et présent dans le gel sous forme moins active.

7. Dispositif selon l'une quelconque des revendications précédentes, **caractérisé en ce que** les moyens (1, 84) pour contenir le gel sont associés à une source lumineuse issue de lumière halogène, à arc ou par LED (19), conduite par une fibre (18).

8. Dispositif selon l'une quelconque des revendications 1 à 6, **caractérisé en ce que** les moyens (1, 84) pour contenir le gel (25) incorporent des diodes (19) électroluminescentes (LEDS) générant une source lumineuse.

9. Dispositif selon l'une quelconque des revendications précédentes, **caractérisé en ce qu'**il comporte, à l'intérieur des moyens (1, 84) pour contenir le gel, des moyens de contrôle de l'évolution électrique ou thermique du traitement de blanchiment, se présentant sous la forme de capteurs (21, 12).

10. Dispositif selon la revendication 9, **caractérisé en ce que** les moyens de contrôle consistent en un spectrocolorimètre ou un colorimètre composé d'un générateur de lumière (39) et d'un analyseur différentiel (47) capable de faire une étude comparative entre la valeur de la source (36) et la valeur s'étant réfléchie sur l'objet (37).

11. Dispositif selon l'une quelconque des revendications précédentes, **caractérisé en ce que** les courants électriques, électromagnétiques ou électrophorétiques utilisés pour le déplacement des molécules sont de profil et de modulation variable en tension, fréquence et amplitude.

12. Dispositif selon l'une quelconque des revendications précédentes, **caractérisé en ce que** le gel électrophorétique (25) de base permettant le passage du courant électrique, électromagnétique ou électrophorétique contient des molécules responsables de l'action de blanchiment chargées électriquement afin qu'elles se déplacent au travers du gel vers le corps à traiter sous l'action du courant utilisé.

13. Dispositif selon l'une quelconque des revendications précédentes, **caractérisé en ce que** le gel électrophorétique (25) de base permettant le passage du courant électrique, électromagnétique ou électrophorétique contient des molécules responsables de l'action de renforcement du corps à traiter chargées électriquement afin qu'elle se déplace au travers du gel vers le corps à traiter sous l'action du courant utilisé et quelle compense les éventuelles dégradations occasionnées par l'action de blanchiment.

14. Dispositif selon l'une quelconque des revendications précédentes, **caractérisé en ce que** le gel électrophorétique (25) de base permettant le passage du courant électrique, électromagnétique ou électrophorétique est chargé électriquement permettant ainsi par son mouvement propre d'entraîner les molécules responsables de l'action de blanchiment ou de renforcement qui ne seraient pas chargées électriquement afin qu'elle se déplace passivement vers le corps sous l'action du déplacement du gel.

15. Dispositif selon l'une quelconque des revendications précédentes, **caractérisé en ce qu'**il comporte un bouton (6) pour inverser la polarité des électrodes responsables de la création des champs électriques, électrophorétique et électromagnétiques utilisé pour le déplacement du gel ou des molécules responsables dans le processus de blanchiment.

16. Dispositif selon l'une quelconque des revendications précédentes, **caractérisé en ce que** les moyens thermiques pour l'activation des molécules mises en jeux dans le processus de blanchiment et déplacées par le courant électrophorétique, électromagnétique ou électrique, consistent en une résistance électrique (2), une émission photonique (19, 20) ou un fluide en circulation (66).

17. Dispositif selon l'une quelconque des revendications précédentes, **caractérisé en ce que** le gel (25) est prévu apte à changer de couleur en fonction de l'évolution de la réaction de blanchiment, du degré d'activation des molécules responsables du blanchiment ou du pourcentage de molécules responsable de la coloration retirées du corps et présentent dans ledit gel (25).

## Claims

1. Bleaching device using electro-optical and chemical means, in particular in the medical and dental field, for bleaching parts of the body such as the teeth, the hairs or the nails, **characterised in that** it comprises:
- an electric, electrophoretic or electromagnetic wave-current generator/controller (4) with a varying profile and/or modulation,
- means (3) for transmitting this current to said parts to be bleached,
- means for creating a non-traumatizing electric, electrophoretic or electromagnetic field (2, 26, 29, 49) through the treated area, comprising electrodes and a conductive gel (25),
- means (1, 84) for containing said gel (25) in contact with the poles and body, allowing the continuity of this field,
- active products (25) used for bleaching, sensitive to the electrophoretic currents,
- means for activating said products either by means of light (19) or by means of heat (27).

2. Device according to claim 1, **characterised in that** it includes a central unit (8, 9, 10) for control, storage and direct monitoring by the operator or indirect monitoring by the memory or over the Internet of the parameters of operation of the electric, electrophoretic or electromagnetic source, in order to allow to control and simplify the penetration of all or part of the molecules sensitive to the electric, electrophoretic or electromagnetic field, active for the bleaching and the protection of the part target of the treatment and the exit of all or part of the molecules sensitive to the electric, electrophoretic or electromagnetic field and which are undesirable or have reacted in this bleaching process and can be removed from the body, but also means for direct control by feedback at the central unit, or manual control by the operator of the parameters allowing to know the evolution and the results of the bleaching action carried out, as are the thermal or electric variations (21) and the modification in coloration (12) induced by the treatment.

3. Device according to claim 1 or claim 2, **characterised in that** it includes an electrophoretic current generator/controller (4), a conduction circuit (3) and a resistor creating a pole in the means (1, 84) for containing the gel (25), the second pole being in contact with a portion of the body.

4. Device according to any one of the preceding claims, **characterised in that** the means (1, 84) for containing the gel are modelled (49) on the portion of the patient's body.

5. Device according to any one of claims 1 to 3, **characterised in that** the second pole located in contact with the body is adapted to be placed in a tube containing an electrophoretic bleaching gel as the root (81) of a devitalized tooth (82).

6. Device according to any one of the preceding claims, **characterised in that** it includes a photonic activator (19) capable of activating the products (25) used for bleaching and present in the gel in a less active form.

7. Device according to any one of the preceding claims, **characterised in that** the means (1, 84) for containing the gel are associated with a source of light proceeding from halogen, arc light or from LEDs (19), transmitted by means of a fiber (18).

8. Device according to any one of claims 1 to 6, **characterised in that** the means (1, 84) for containing the gel (25) incorporate light-emitting diodes (19) (LEDs) generating a source of light.

9. Device according to any one of the preceding claims, **characterised in that** it includes, inside the means (1, 84) for containing the gel, means for controlling the electric or thermal evolution of the bleaching treatment, in the form of sensors (21, 12).

10. Device according to claim 9, **characterised in that** the means for controlling consist of a spectrocolorimeter or a colorimeter comprised of a light generator (39) and of a differential analyzer (47) capable of carrying out a comparative study between the value of the source (36) and the value being reflected onto the object (37).

11. Device according to any one of the preceding claims, **characterised in that** the electrical, electromagnetic or electrophoretic currents used for the displacement of the molecules have a varying profile and modulation in tension, frequency and amplitude.

12. Device according to any one of the preceding claims, **characterised in that** the basic electrophoretic gel (25) allowing the passing through of the electrical, electromagnetic or electrophoretic current contains electrically charged molecules responsible for the bleaching action, so that they move through the gel towards the body to be treated under the action of the current used.

13. Device according to any one of the preceding claims, **characterised in that** the basic electrophoretic gel (25) allowing the passing through of the electrical, electromagnetic or electrophoretic current contains electrically charged molecules responsible for the action of reinforcement of the body to be treated, so that they move through the gel towards the body to be treated under the action of the current used and that they compensate for the possible degradations caused by the bleaching action.

14. Device according to any one of the preceding claims, **characterised in that** basic electrophoretic gel (25) allowing the passing through of the electrical, electromagnetic or electrophoretic current is electrically charged, thus allowing, by its own movement, to drag the molecules responsible for the bleaching or reinforcing action which would not be electrically charged, so that they move passively towards the body under the action of the displacement of the gel.

15. Device according to any one of the preceding claims, **characterised in that** it includes a button (6) for reversing the polarity of the electrodes responsible for creating the electric, electrophoretic and electromagnetic fields used for the displacement of the gel or of the molecules responsible in the bleaching process.

16. Device according to any one of the preceding claims, **characterised in that** thermal means for the activation of the molecules used in the process of bleaching and moved by the electrophoretic, electromagnetic or electric current consist of an electric resistor (2), a photonic emission (19, 20) or a circulating fluid (66).

17. Device according to any one of the preceding claims, **characterised in that** the gel (25) is designed capable of changing color according to the evolution of the bleaching reaction, the degree of activation of the molecules responsible for the bleaching or the percentage of molecules responsible for coloration removed from the body and present in said gel (25).

## Patentansprüche

1. Vorrichtung zum Bleichen unter Verwendung von elektrooptischen und chemischen Mitteln, insbesondere im Medizin- und Zahnmedizinbereich im Hinblick auf Aufhellungen der Partien des Körpers wie den Zähnen, den Haaren oder den Nägeln, **dadurch gekennzeichnet, daß** sie umfaßt:
- einen Stromerzeuger/-regler (4) von einer elektrischen, elektrophoretischen oder elektromagnetischen Welle mit variablem Profil und/oder variabler Modulation,
- Mittel (3), um den Strom zu den aufzuhellenden Partien zu übertragen,
- Mittel, um ein elektrisches, elektrophoretisches oder elektromagnetisches nicht traumatisierendes Feld (2, 26, 29, 49) über der behandelten Zone zu erzeugen, umfassend Elektroden und ein Leitergel (25),
- in Kontakt mit Polen und dem Körper befindliche Mittel (1, 84) zum Enthalten des Gels (25), um die Kontinuität dieses Feldes erlauben,
- aktive Produkte (25), die zum Bleichen eingesetzt werden und sensibel gegenüber den elektrophoretischen Strömen sind,
- Mittel, um die Produkte zu aktivieren, entweder durch Licht (19) oder durch Wärme (27).

2. Vorrichtung nach Anspruch 1, **dadurch gekennzeichnet, daß** sie eine Zentraleinheit (8, 9, 10) zur Verwaltung, zur Speicherung und zur direkten Regelung durch den Anwender oder indirekt durch den Speicher oder das Internet von Funktionsparametern der elektrischen, elektrophoretischen oder elektromagnetischen Quelle umfaßt, um es zuzulassen, die Penetrierung von allen oder Teilen der Moleküle zu beherrschen, die gegenüber dem elektrischen, elektrophoretischen oder elektromagnetischen Feld empfindlich sind, aktiviert für das Bleichen und den Schutz der Zielpartie der Behandlung und den Austritt von allen oder Teilen der Moleküle, die empfindlich sind gegenüber dem elektrischen, elektrophoretischen oder elektromagnetischen Feld und ungewünscht sind oder nicht reagiert haben in dem Aufhellungsverfahren und vom Körper abgezogen werden können, aber auch Mittel zur direkten Regelung durch Feedback auf der Zentraleinheit oder manuell durch den Betreiber der Parameter, die es erlauben, die Entwicklung und die Ergebnisse des ausgeführten Weisungsvorgangs wie die thermischen oder elektrischen Variationen (21) und die Modifikation der Färbung (12) zu kennen, die durch die Behandlung ausgelöst werden.

3. Vorrichtung nach Anspruch 1 oder Anspruch 2, **dadurch gekennzeichnet, daß** sie einen Erzeuger/Regler (4) von elektrophoretischem Strom, einen Leiterkreis (3) und einen Widerstand hat, der einen Pol in den Mitteln (1, 84) zum Enthalten des Gels (25) erzeugt, wobei der zweite Pol in Kontakt mit einer Körperpartie ist.

4. Vorrichtung nach einem der vorangehenden Ansprüche, **dadurch gekennzeichnet, daß** die Mittel (1, 84) zum Enthalten des Gels moduliert sind (49) auf dem Teil des Körpers des Patienten.

5. Vorrichtung nach einem der Ansprüche 1 bis 3, **dadurch gekennzeichnet, daß** der zweite Pol in Kontakt mit dem Körper angeordnet ist und ausgelegt ist, um sich in einer Röhre zu plazieren, die ein elektrophoretisches Gel zum Bleichen enthält, wie der Wurzel (81) eines devitalisierten Zahns (82).

6. Vorrichtung nach einem der vorhergehenden Ansprüche, **dadurch gekennzeichnet, daß** sie einen photonischen Aktivator (19) umfaßt, der geeignet ist, die Produkte (25) zu aktivieren, die zum Bleichen verwendet werden und in dem Gel in weniger aktiver Form vorliegen.

7. Vorrichtung nach einem der vorhergehenden Ansprüche, **dadurch gekennzeichnet, daß** die Mittel (1, 84) zum Enthalten des Gels verbunden sind mit einer Lichtquelle aus Halogenlicht, Bogenlicht oder LED-Licht (19), das durch eine Faser (18) geleitet wird.

8. Vorrichtung nach einem der Ansprüche 1 bis 6 **dadurch gekennzeichnet, daß** die Mittel (1, 84) zum Enthalten des Gels (25) elektrolumineszente Dioden (19) (LEDS) haben, die eine Lichtquelle erzeugen.

9. Vorrichtung nach einem der vorhergehenden Ansprüche, **dadurch gekennzeichnet, daß** sie innen Mittel (1, 84) zum Enthalten des Gels und Mittel zur Regelung der Entwicklung von Elektrizität oder Wärme der Bleichungsbehandlung umfaßt, die in Form von Sensoren (21, 12) vorliegen.

10. Vorrichtung nach Anspruch 9, **dadurch gekennzeichnet, daß** die Regelungsmittel in einem Spektrocolorimeter oder einem Colorimeter bestehen, das besteht aus einem Lichterzeuger (39) und einem Differentialanalysator (47), der in der Lage ist, eine vergleichbare Untersuchung zwischen dem Wert der Quelle (36) und dem auf dem Gegenstand (37) reflektierten Wert durchzuführen

11. Vorrichtung nach einem der vorhergehenden Ansprüche, **dadurch gekennzeichnet, daß** die elektrischen, elektromagnetischen oder elektrophoretischen Ströme, die für die Bewegung der Moleküle verwendet werden, mit einem Profil oder einer Modulation ist, die an Spannung, Frequenz und Amplitude variabel sind.

12. Vorrichtung nach einem der vorhergehenden Ansprüche, **dadurch gekennzeichnet, daß** das elektrophoretische Basisgel (25), welches den Durchgang des elektrischen, elektromagnetischen oder elektrophoretischen Stromes erlaubt, Moleküle enthält, die für die Bleichungswirkung verantwortlich sind und elektrisch aufgeladen sind, damit sie sich durch das Gel zu dem umzuwandelnden Körper unter der Wirkung des verwendeten Stroms bewegen.

13. Vorrichtung nach einem der vorhergehenden Ansprüche, **dadurch gekennzeichnet, daß** das elektrophoretische Basisgel (25), das den Durchgang des elektrischen, elektromagnetischen oder elektrophoretischen Stromes erlaubt, Moleküle enthält, die für die Verstärkungswirkung des zu behandelnden Körpers verantwortlich und elektrisch geladen sind, damit es sich über das Gel zum zu behandelnden Körper unter der Wirkung des verwendeten Stromes bewegt und welches die eventuellen durch die Weißungswirkung hervorgerufenen Beschädigungen ausgleicht.

14. Vorrichtung nach einem der vorhergehenden Ansprüche, **dadurch gekennzeichnet, daß** das elektrophoretische Basisgel (25), das den Durchgang des elektrischen, elektromagnetischen oder elektrophoretischen Stroms zuläßt, elektrisch geladen ist, was es so durch seine eigene Bewegung erlaubt, die Moleküle mitzuziehen, die für die Bleichungswirkung oder Verstärkungswirkung verantwortlich sind und die nicht elektrisch geladen sind, damit sie sich passiv zum Körper unter der Wirkung der Bewegung des Gels verschiebt.

15. Vorrichtung nach einem der vorhergehenden Ansprüche, **dadurch gekennzeichnet, daß** sie einen Knopf umfaßt, um die Polarität der Elektroden umzukehren, die für die Erzeugung der elektrischen, elektrophoretischen und elektromagnetischen Felder verantwortlich sind, verwendet für die Bewegung des Gels oder der Moleküle, die in dem Bleichungsverfahren verantwortlich sind.

16. Vorrichtung nach einem der vorhergehenden Ansprüche, **dadurch gekennzeichnet, daß** die thermischen Mittel zur Aktivierung der in dem Verfahren zum Bleichen eingesetzten Moleküle und welche durch den elektrophoretischen, elektromagnetischen oder elektrischen Strom bewegt werden, bestehen aus einem elektrischen Widerstand (2) einer Photonenemission (19, 20) oder einem Fluid in Zirkulation (66).

17. Vorrichtung nach einem der der vorhergehenden Ansprüche, **dadurch gekennzeichnet, daß** das Gel (25) als dazu geeignet vorgesehen ist, die Farbe in Abhängigkeit der Entwicklung der Bleichreaktion, dem Aktivierungsgrad der Moleküle, die für das Bleichen verantwortlich sind, oder des Prozentanteils von Molekülen, die für die Färbung verantwortlich sind und vom Körper zurückgehalten werden und in dem Gel (25) vorliegen, zu ändern.
